# EUROPEAN PATENT APPLICATION

(11) **EP 4 628 118 A1**
(43) Date of publication of application: **08.10.2025**
(21) Application number: 24741527.6
(22) Date of filing: 10.01.2024
(51) Int. Cl.: A61M 1/16, A61M 60/37

(54) **BLOOD PURIFICATION DEVICE**

(30) Priority: 12.01.2023 JP 2023002862
(71) Applicant: Nipro Corporation, Osaka 566-8510 (JP)
(72) Inventor: KANAGAWA, Takaiki, Chuo-shi, Yamanashi 409-3801 (JP); MURAKAMI, Masaki, Chuo-shi, Yamanashi 409-3801 (JP); ISHII, Hiroshi, Settsu-shi, Osaka 566-8510 (JP); NAKAMURA, Shota, Settsu-shi, Osaka 566-8510 (JP); SASAGAWA, Mitsumasa, Settsu-shi, Osaka 566-8510 (JP)
(74) Representative: Prüfer & Partner mbB Patentanwälte · Rechtsanwälte
(86) International application number: PCT/JP2024/000249
(87) International publication number: WO 2024/150754

(57) **Abstract**

In a first step (S1), a controller actually measures a substantial replacement fluid flow rate of a replacement fluid pump from weight change measured by a scale and calculates a replacement fluid correction value which is a difference between a set replacement fluid flow rate and the substantial replacement fluid flow rate. In a second step (S2), the controller actually measures a substantial dialysis fluid flow rate of a dialysis fluid pump from weight change measured by the scale and calculates a dialysis fluid correction value which is a difference between a set dialysis fluid flow rate and the substantial dialysis fluid flow rate. In a third step (S3), the controller actually measures a difference between a substantial waste fluid flow rate of a waste fluid pump and the substantial dialysis fluid flow rate of the dialysis fluid pump from weight change measured by the scale, calculates the substantial waste fluid flow rate from this difference and the substantial dialysis fluid flow rate actually measured in the second step, and calculates a waste fluid correction value which is a difference between a set waste fluid flow rate and the substantial waste fluid flow rate.

## Description

### TECHNICAL FIELD

The present invention relates to a blood purification apparatus.

### BACKGROUND ART

Prior art documents that disclose a configuration of a flow rate calibration device of a fluid replacement pump in a hemodialyzer include Japanese Patent Laying-Open No. 2020-81337 (PTL 1). The flow rate calibration device of the fluid replacement pump in the hemodialyzer described in PTL 1 includes a dialysate chamber, a dialysate supply passage, a dialysate recovery passage, a dialysate pump, a blood circuit, a fluid replacement passage, a fluid replacement pump, and control means. The dialysate chamber is divided by a diaphragm into a supply chamber and a recovery chamber. The dialysate supply passage connects the supply chamber and a dialyzer to each other. The dialysate recovery passage connects the dialyzer and the recovery chamber to each other. The dialysate pump is provided in the dialysate recovery passage. The blood circuit is connected to the dialyzer. The fluid replacement passage is connected to a dialysate passage and the blood circuit. The fluid replacement pump is provided in the fluid replacement passage. The control means controls the dialysate pump and the fluid replacement pump.

### CITATION LIST

### PATENT LITERATURE

PTL 1: Japanese Patent Laying-Open No. 2020-81337

### SUMMARY OF INVENTION

### TECHNICAL PROBLEM

In the flow rate calibration device of the fluid replacement pump in the hemodialyzer described in PTL 1, the flow rate of the fluid replacement pump is regulated during dialysis therapy. Therefore, at the time of start of the therapy, a substantial flow rate of each of the fluid replacement pump, the dialysate pump, and a waste fluid pump may be displaced from a set flow rate.

The present invention was made in view of the problem above, and an object thereof is to provide a blood purification apparatus in which each of a replacement fluid pump, a dialysis fluid pump, and a waste fluid pump can be driven at an accurate flow rate at the time of start of therapy.

### SOLUTION TO PROBLEM

A blood purification apparatus based on a first aspect of the present invention includes a blood purification instrument, an artery-side blood circuit, a vein-side blood circuit, a replacement fluid pipe channel, a dialysis fluid pipe channel, a waste fluid pipe channel, a blood pump, a replacement fluid pump, a dialysis fluid pump, a waste fluid pump, an artery-side on-off valve, a priming fluid pipe channel, a first fluid storage portion, a second fluid storage portion, a scale, and a controller. The artery-side blood circuit is connected to the blood purification instrument and provided for inflow of blood into the blood purification instrument. The vein-side blood circuit is connected to the blood purification instrument and provided for outflow of blood from the blood purification instrument. The replacement fluid pipe channel is connected to the artery-side blood circuit or the vein-side blood circuit, a replacement fluid being supplied therethrough. Through the dialysis fluid pipe channel, a dialysis fluid is supplied to the blood purification instrument. Through the waste fluid pipe channel, a waste fluid discharged from the blood purification instrument flows. The blood pump is provided in the artery-side blood circuit and delivers blood. The replacement fluid pump is provided in the replacement fluid pipe channel and delivers the replacement fluid. The dialysis fluid pump is provided in the dialysis fluid pipe channel and delivers the dialysis fluid. The waste fluid pump is provided in the waste fluid pipe channel and delivers the waste fluid. The artery-side on-off valve is provided in the artery-side blood circuit and opens and closes the artery-side blood circuit. The priming fluid pipe channel is connected to the artery-side blood circuit between the artery-side on-off valve and the blood pump, a priming fluid being supplied therethrough. The first fluid storage portion is connected to the replacement fluid pipe channel and the dialysis fluid pipe channel, and in the first fluid storage portion, the replacement fluid and the dialysis fluid are temporarily stored, and from the first fluid storage portion, the stored replacement fluid and the stored dialysis fluid can be delivered. The second fluid storage portion is connected to the waste fluid pipe channel, and in the second fluid storage portion, the waste fluid is temporarily stored, and from the second fluid storage portion, the stored waste fluid can be delivered. The scale can measure weight change of the first fluid storage portion and the second fluid storage portion as a whole. The controller controls drive of each of the blood pump, the replacement fluid pump, the dialysis fluid pump, and the waste fluid pump. The controller performs a first step, a second step, and a third step. In the first step, the controller actually measures a substantial replacement fluid flow rate of the replacement fluid pump from weight change measured by the scale by activating the replacement fluid pump at a set replacement fluid flow rate for a set time period to supply the replacement fluid from the first fluid storage portion while the replacement fluid is stored in the first fluid storage portion during priming in which the artery-side on-off valve is closed and the priming fluid is supplied from the priming fluid pipe channel to the artery-side blood circuit, the blood purification instrument, and the vein-side blood circuit, and calculates a replacement fluid correction value which is a difference between the set replacement fluid flow rate and the substantial replacement fluid flow rate. In the second step, the controller actually measures a substantial dialysis fluid flow rate of the dialysis fluid pump from weight change measured by the scale by activating the dialysis fluid pump at a set dialysis fluid flow rate for a set time period to supply the dialysis fluid from the first fluid storage portion while the dialysis fluid is stored in the first fluid storage portion and activating the waste fluid pump at a set waste fluid flow rate for the set time period to discharge the waste fluid through the waste fluid pipe channel during the priming, and calculates a dialysis fluid correction value which is a difference between the set dialysis fluid flow rate and the substantial dialysis fluid flow rate. In the third step, the controller actually measures a difference between a substantial waste fluid flow rate of the waste fluid pump and the substantial dialysis fluid flow rate of the dialysis fluid pump from weight change measured by the scale by activating the dialysis fluid pump at the set dialysis fluid flow rate for the set time period to supply the dialysis fluid from the first fluid storage portion while the dialysis fluid is stored in the first fluid storage portion and activating the waste fluid pump at a set waste fluid flow rate for this set time period to store the waste fluid in the second fluid storage portion during the priming, calculates the substantial waste fluid flow rate from this difference and the substantial dialysis fluid flow rate actually measured in the second step, and calculates a waste fluid correction value which is a difference between the set waste fluid flow rate and the substantial waste fluid flow rate.

A blood purification apparatus based on a second aspect of the present invention includes a blood purification instrument, an artery-side blood circuit, a vein-side blood circuit, a replacement fluid pipe channel, a dialysis fluid pipe channel, a waste fluid pipe channel, a blood pump, a replacement fluid pump, a dialysis fluid pump, a waste fluid pump, an artery-side on-off valve, a priming fluid pipe channel, a first fluid storage portion, a second fluid storage portion, a scale, and a controller. The artery-side blood circuit is connected to the blood purification instrument and provided for inflow of blood into the blood purification instrument. The vein-side blood circuit is connected to the blood purification instrument and provided for outflow of blood from the blood purification instrument. The replacement fluid pipe channel is connected to the artery-side blood circuit or the vein-side blood circuit, a replacement fluid being supplied therethrough. Through the dialysis fluid pipe channel, a dialysis fluid is supplied to the blood purification instrument. Through the waste fluid pipe channel, a waste fluid discharged from the blood purification instrument flows. The blood pump is provided in the artery-side blood circuit and delivers blood. The replacement fluid pump is provided in the replacement fluid pipe channel and delivers the replacement fluid. The dialysis fluid pump is provided in the dialysis fluid pipe channel and delivers the dialysis fluid. The waste fluid pump is provided in the waste fluid pipe channel and delivers the waste fluid. The artery-side on-off valve is provided in the artery-side blood circuit and opens and closes the artery-side blood circuit. The priming fluid pipe channel is connected to the artery-side blood circuit between the artery-side on-off valve and the blood pump, a priming fluid being supplied therethrough. The first fluid storage portion is connected to the replacement fluid pipe channel and the dialysis fluid pipe channel, and in the first fluid storage portion, the replacement fluid and the dialysis fluid are temporarily stored, and from the first fluid storage portion, the stored replacement fluid and the stored dialysis fluid can be delivered. The second fluid storage portion is connected to the waste fluid pipe channel, and in the second fluid storage portion, the waste fluid is temporarily stored, and from the second fluid storage portion, the stored waste fluid can be delivered. The scale can measure weight change of the first fluid storage portion and the second fluid storage portion as a whole. The controller controls drive of each of the blood pump, the replacement fluid pump, the dialysis fluid pump, and the waste fluid pump. The controller performs a first step, a second step, and a third step. In the first step, the controller actually measures a substantial replacement fluid flow rate of the replacement fluid pump from weight change measured by the scale by activating the replacement fluid pump at a set replacement fluid flow rate for a set time period to supply the replacement fluid from the first fluid storage portion while the replacement fluid is stored in the first fluid storage portion during priming in which the artery-side on-off valve is closed and the priming fluid is supplied from the priming fluid pipe channel to the artery-side blood circuit, the blood purification instrument, and the vein-side blood circuit, and calculates a replacement fluid correction value which is a difference between the set replacement fluid flow rate and the substantial replacement fluid flow rate. In the second step, the controller actually measures a substantial dialysis fluid flow rate of the dialysis fluid pump from weight change measured by the scale by activating the dialysis fluid pump at a set dialysis fluid flow rate for a set time period to supply the dialysis fluid from the first fluid storage portion while the dialysis fluid is stored in the first fluid storage portion and activating the waste fluid pump at a set waste fluid flow rate for this set time period to discharge the waste fluid through the waste fluid pipe channel during the priming, and calculates a dialysis fluid correction value which is a difference between the set dialysis fluid flow rate and the substantial dialysis fluid flow rate. In the third step, the controller actually measures a substantial waste fluid flow rate of the waste fluid pump from weight change measured by the scale by activating the dialysis fluid pump at the set dialysis fluid flow rate for the set time period to supply the dialysis fluid through the dialysis fluid pipe channel without going through the first fluid storage portion and activating the waste fluid pump at the set waste fluid flow rate for this set time period to store the waste fluid in the second fluid storage portion during the priming, and calculates a waste fluid correction value which is a difference between the set waste fluid flow rate and the substantial waste fluid flow rate.

In one form of the present invention, the controller activates the blood pump to supply the priming fluid to the blood purification instrument when the controller performs the second step and the third step.

A blood purification apparatus based on a third aspect of the present invention includes a blood purification instrument, an artery-side blood circuit, a vein-side blood circuit, a replacement fluid pipe channel, a dialysis fluid pipe channel, a waste fluid pipe channel, a blood pump, a replacement fluid pump, a dialysis fluid pump, a waste fluid pump, an artery-side on-off valve, a priming fluid pipe channel, a first fluid storage portion, a second fluid storage portion, a scale, and a controller. The artery-side blood circuit is connected to the blood purification instrument and provided for inflow of blood into the blood purification instrument. The vein-side blood circuit is connected to the blood purification instrument and provided for outflow of blood from the blood purification instrument. The replacement fluid pipe channel is connected to the artery-side blood circuit or the vein-side blood circuit, a replacement fluid being supplied therethrough. Through the dialysis fluid pipe channel, a dialysis fluid is supplied to the blood purification instrument. Through the waste fluid pipe channel, a waste fluid discharged from the blood purification instrument flows. The blood pump is provided in the artery-side blood circuit and delivers blood. The replacement fluid pump is provided in the replacement fluid pipe channel and delivers the replacement fluid. The dialysis fluid pump is provided in the dialysis fluid pipe channel and delivers the dialysis fluid. The waste fluid pump is provided in the waste fluid pipe channel and delivers the waste fluid. The artery-side on-off valve is provided in the artery-side blood circuit and opens and closes the artery-side blood circuit. The priming fluid pipe channel is connected to the artery-side blood circuit between the artery-side on-off valve and the blood pump, a priming fluid being supplied therethrough. The first fluid storage portion is connected to the replacement fluid pipe channel and the dialysis fluid pipe channel, and in the first fluid storage portion, the replacement fluid and the dialysis fluid are temporarily stored, and from the first fluid storage portion, the stored replacement fluid and the stored dialysis fluid can be delivered. The second fluid storage portion is connected to the waste fluid pipe channel, and in the second fluid storage portion, the waste fluid is temporarily stored, and from the second fluid storage portion, the stored waste fluid can be delivered. The scale can measure weight change of the first fluid storage portion and the second fluid storage portion as a whole. The controller controls drive of each of the blood pump, the replacement fluid pump, the dialysis fluid pump, and the waste fluid pump. The controller performs a first step, a second step, and a third step. In the first step, the controller actually measures a substantial replacement fluid flow rate of the replacement fluid pump from weight change measured by the scale by activating the replacement fluid pump at a set replacement fluid flow rate for a set time period to supply the replacement fluid from the first fluid storage portion while the replacement fluid is stored in the first fluid storage portion during priming in which the artery-side on-off valve is closed and the priming fluid is supplied from the priming fluid pipe channel to the artery-side blood circuit, the blood purification instrument, and the vein-side blood circuit, and calculates a replacement fluid correction value which is a difference between the set replacement fluid flow rate and the substantial replacement fluid flow rate. In the second step, the controller actually measures a substantial dialysis fluid flow rate of the dialysis fluid pump from weight change measured by the scale by activating the dialysis fluid pump at a set dialysis fluid flow rate for a set time period to supply the dialysis fluid from the first fluid storage portion while the dialysis fluid is stored in the first fluid storage portion and activating the waste fluid pump at a set waste fluid flow rate for this set time period to discharge the waste fluid through the waste fluid pipe channel during the priming, and calculates a dialysis fluid correction value which is a difference between the set dialysis fluid flow rate and the substantial dialysis fluid flow rate. In the third step, the controller actually measures a substantial waste fluid flow rate of the waste fluid pump from weight change measured by the scale by activating the blood pump to discharge the waste fluid from the blood purification instrument to the waste fluid pipe channel and activating the waste fluid pump at the set waste fluid flow rate for this set time period to store the waste fluid in the second fluid storage portion during the priming, and calculates a waste fluid correction value which is a difference between the set waste fluid flow rate and the substantial waste fluid flow rate.

A blood purification apparatus based on a fourth aspect of the present invention includes a blood purification instrument, an artery-side blood circuit, a vein-side blood circuit, a replacement fluid pipe channel, a dialysis fluid pipe channel, a waste fluid pipe channel, a blood pump, a replacement fluid pump, a dialysis fluid pump, a waste fluid pump, an artery-side on-off valve, a priming fluid pipe channel, a first fluid storage portion, a second fluid storage portion, a scale, and a controller. The artery-side blood circuit is connected to the blood purification instrument and provided for inflow of blood into the blood purification instrument. The vein-side blood circuit is connected to the blood purification instrument and provided for outflow of blood from the blood purification instrument. The replacement fluid pipe channel is connected to the artery-side blood circuit or the vein-side blood circuit, a replacement fluid being supplied therethrough. Through the dialysis fluid pipe channel, a dialysis fluid is supplied to the blood purification instrument. Through the waste fluid pipe channel, a waste fluid discharged from the blood purification instrument flows. The blood pump is provided in the artery-side blood circuit and delivers blood. The replacement fluid pump is provided in the replacement fluid pipe channel and delivers the replacement fluid. The dialysis fluid pump is provided in the dialysis fluid pipe channel and delivers the dialysis fluid. The waste fluid pump is provided in the waste fluid pipe channel and delivers the waste fluid. The artery-side on-off valve is provided in the artery-side blood circuit and opens and closes the artery-side blood circuit. The priming fluid pipe channel is connected to the artery-side blood circuit between the artery-side on-off valve and the blood pump, a priming fluid being supplied therethrough. The first fluid storage portion is connected to the replacement fluid pipe channel and the dialysis fluid pipe channel, and in the first fluid storage portion, the replacement fluid and the dialysis fluid are temporarily stored, and from the first fluid storage portion, the stored replacement fluid and the stored dialysis fluid can be delivered. The second fluid storage portion is connected to the waste fluid pipe channel, and in the second fluid storage portion, the waste fluid is temporarily stored, and from the second fluid storage portion, the stored waste fluid can be delivered. The scale can measure weight change of the first fluid storage portion and the second fluid storage portion as a whole. The controller controls drive of each of the blood pump, the replacement fluid pump, the dialysis fluid pump, and the waste fluid pump. The controller performs a first step, a second step, and a third step. In the first step, the controller actually measures a first flow rate from weight change measured by the scale by activating the waste fluid pump at a set waste fluid flow rate for a set time period to store the waste fluid in the second fluid storage portion while activating the replacement fluid pump at a set replacement fluid flow rate and the dialysis fluid pump at a set dialysis fluid flow rate for the set time period to supply the replacement fluid and the dialysis fluid from the first fluid storage portion while the replacement fluid and the dialysis fluid are stored in the first fluid storage portion during priming in which the artery-side on-off valve is closed and the priming fluid is supplied from the priming fluid pipe channel to the artery-side blood circuit, the blood purification instrument, and the vein-side blood circuit. In the second step, the controller changes any one of the set replacement fluid flow rate, the set dialysis fluid flow rate, and the set waste fluid flow rate that have been set in the first step and actually measures a second flow rate from weight change measured by the scale by activating the replacement fluid pump, the dialysis fluid pump, and the waste fluid pump for the set time period to store the waste fluid in the second fluid storage portion while supplying the replacement fluid and the dialysis fluid from the first fluid storage portion. In the third step, the controller changes any another one of the set replacement fluid flow rate, the set dialysis fluid flow rate, and the set waste fluid flow rate that have been set in the first step and actually measures a third flow rate from weight change measured by the scale by activating the replacement fluid pump, the dialysis fluid pump, and the waste fluid pump for the set time period to store the waste fluid in the second fluid storage portion while supplying the replacement fluid and the dialysis fluid from the first fluid storage portion. The controller calculates a first correction value which is a difference between a first substantial flow rate and the any one of the set replacement fluid flow rate, the set dialysis fluid flow rate, and the set waste fluid flow rate, the first substantial flow rate being calculated by dividing a difference calculated by subtracting the first flow rate from the second flow rate by a rate of change in the any one of the set replacement fluid flow rate, the set dialysis fluid flow rate, and the set waste fluid flow rate. The controller calculates a second correction value which is a difference between a second substantial flow rate and the any another one of the set replacement fluid flow rate, the set dialysis fluid flow rate, and the set waste fluid flow rate, the second substantial flow rate being calculated by dividing a difference calculated by subtracting the first flow rate from the third flow rate by a rate of change in the any another one of the set replacement fluid flow rate, the set dialysis fluid flow rate, and the set waste fluid flow rate. The controller calculates a third correction value which is a difference between a third substantial flow rate and remaining one of the set replacement fluid flow rate, the set dialysis fluid flow rate, and the set waste fluid flow rate that have been set in the first step, the third substantial flow rate being calculated in accordance with a relational expression of the first flow rate with the first substantial flow rate and the second substantial flow rate.

In one form of the present invention, the controller activates the blood pump to supply the priming fluid to the blood purification instrument when the controller performs the first step to the third step.

### ADVANTAGEOUS EFFECTS OF INVENTION

According to the present invention, each of the replacement fluid pump, the dialysis fluid pump, and the waste fluid pump can be driven at an accurate flow rate at the time of start of therapy.

### BRIEF DESCRIPTION OF DRAWINGS

Fig. 1 is a circuit diagram showing a configuration of a blood purification apparatus according to one embodiment of the present invention.
Fig. 2 is a block diagram showing relation of electrical connection between a controller and each component in the blood purification apparatus according to one embodiment of the present invention.
Fig. 3 is a flowchart showing an operation performed by a controller to calculate a correction value of a flow rate of each of a dialysis fluid pump, a replacement fluid pump, and a waste fluid pump during priming in a blood purification apparatus according to a first embodiment of the present invention.
Fig. 4 is a circuit diagram showing a state in which a first step is performed in the blood purification apparatus according to the first embodiment of the present invention.
Fig. 5 is a circuit diagram showing a state in which a second step is performed in the blood purification apparatus according to the first embodiment of the present invention.
Fig. 6 is a circuit diagram showing a state in which a third step is performed in the blood purification apparatus according to the first embodiment of the present invention.
Fig. 7 is a circuit diagram showing a state in which each of the first step to the third step is performed in a blood purification apparatus according to a second embodiment of the present invention.
Fig. 8 is a flowchart showing an operation performed by the controller to calculate a correction value of a flow rate of each of the dialysis fluid pump, the replacement fluid pump, and the waste fluid pump during priming in the blood purification apparatus according to the second embodiment of the present invention.

### DESCRIPTION OF EMBODIMENTS

A blood purification apparatus according to each embodiment of the present invention will be described below with reference to the drawings. In the description of an embodiment below, the same or corresponding elements in the drawings have the same reference characters allotted and description thereof will not be repeated.

In the description of an embodiment below, a blood purification apparatus used for continuous renal replacement therapy (CRRT) will be described as a blood purification apparatus. The blood purification apparatus may be a blood purification apparatus to be used for any of continuous hemodiafiltration (CHDF), continuous hemofiltration (CHF), continuous hemodialysis (CHD), and slow continuous ultrafiltration (SCUF).

### (First Embodiment)

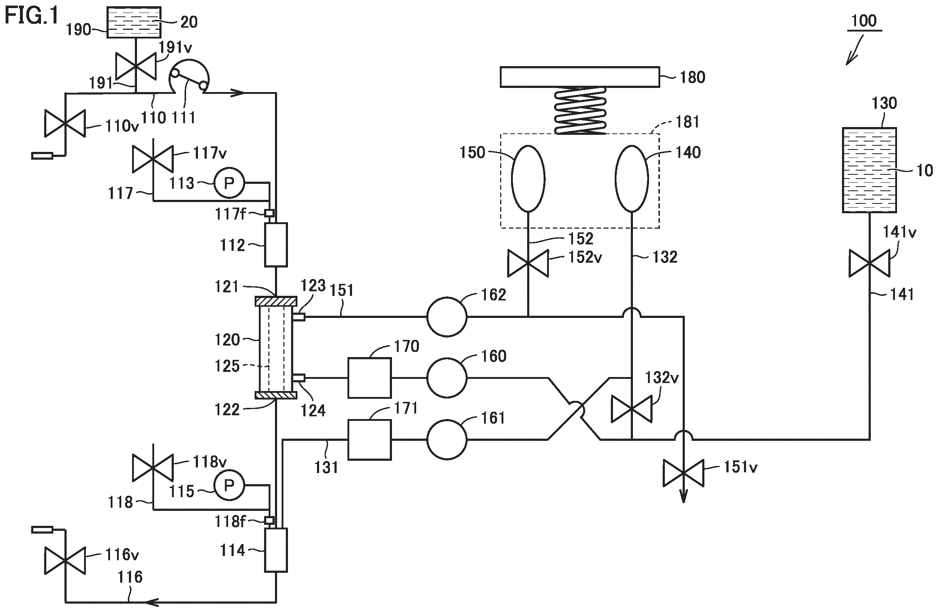

Fig. 1 is a circuit diagram showing a configuration of a blood purification apparatus according to one embodiment of the present invention. As shown in Fig. 1, a blood purification apparatus 100 according to one embodiment of the present invention includes a blood purification instrument 120, an artery-side blood circuit 110, a vein-side blood circuit 116, a replacement fluid pipe channel 131, a dialysis fluid pipe channel 141, a waste fluid pipe channel 151, a blood pump 111, a replacement fluid pump 161, a dialysis fluid pump 160, a waste fluid pump 162, an artery-side on-off valve 110v, a priming fluid pipe channel 191, a first fluid storage portion 140, a second fluid storage portion 150, a scale 180, and a controller.

Blood purification apparatus 100 further includes an artery-side air trap chamber 112, a vein-side air trap chamber 114, a vein-side on-off valve 116v, an artery-side pressure measurement apparatus 113, a vein-side pressure measurement apparatus 115, a first supply source 130, a priming fluid supply source 190, a first connection pipe channel 132, a second connection pipe channel 152, an artery-side gas supply and exhaust pipe channel 117, a vein-side gas supply and exhaust pipe channel 118, a first valve 141v, a second valve 132v, a third valve 151v, a fourth valve 152v, a fifth valve 191v, a sixth valve 117v, a seventh valve 118v, an artery-side protective filter 117f, a vein-side protective filter 118f, a first heater 170, and a second heater 171.

Blood purification instrument 120 contains a semipermeable membrane formed, for example, from a hollow fiber membrane. Blood purification instrument 120 is provided with a blood inlet 121 and a blood outlet 122. Artery-side blood circuit 110 is connected to blood inlet 121. Vein-side blood circuit 116 is connected to blood outlet 122.

Artery-side blood circuit 110 is provided with blood pump 111 that delivers blood. In artery-side blood circuit 110, artery-side air trap chamber 112 is provided between blood pump 111 and blood purification instrument 120. Artery-side air trap chamber 112 is provided with artery-side pressure measurement apparatus 113 that measures a pressure in artery-side air trap chamber 112. Artery-side protective filter 117f is provided between artery-side air trap chamber 112 and artery-side pressure measurement apparatus 113. Artery-side blood circuit 110 is provided with artery-side on-off valve 110v that opens and closes artery-side blood circuit 110.

A pressure of blood taken from an artery of a patient is measured while it flows through artery-side blood circuit 110 and passes through artery-side air trap chamber 112, and thereafter blood flows into blood purification instrument 120 through blood inlet 121. Artery-side air trap chamber 112 is provided to prevent air from being introduced in blood in artery-side blood circuit 110.

Vein-side air trap chamber 114 is provided in vein-side blood circuit 116. Vein-side air trap chamber 114 is provided with vein-side pressure measurement apparatus 115 that measures a pressure in vein-side air trap chamber 114. Vein-side protective filter 118f is provided between vein-side air trap chamber 114 and vein-side pressure measurement apparatus 115. Vein-side blood circuit 116 is provided with vein-side on-off valve 116v that opens and closes vein-side blood circuit 116.

A pressure of blood purified by blood purification instrument 120 is measured while it flows through vein-side blood circuit 116 and passes through vein-side air trap chamber 114, and thereafter blood returns to a vein of the patient. Vein-side air trap chamber 114 is provided to prevent air from being introduced in blood in vein-side blood circuit 116.

Blood purification instrument 120 is further provided with a dialysis fluid inlet 124 and a waste fluid outlet 123. Dialysis fluid pipe channel 141 is connected to dialysis fluid inlet 124. Waste fluid pipe channel 151 is connected to waste fluid outlet 123.

Dialysis fluid pipe channel 141 has an upstream end connected to first supply source 130 that supplies a first fluid 10 which is a replacement fluid and a dialysis fluid. During priming, first supply source 130 supplies a physiological saline as first fluid 10.

Dialysis fluid pipe channel 141 is connected to an upstream end of first connection pipe channel 132. Dialysis fluid pump 160 that delivers the dialysis fluid is connected to dialysis fluid pipe channel 141. Though dialysis fluid pump 160 is a peristaltic pump, it may be a roller pump. The dialysis fluid that has flowed through dialysis fluid pipe channel 141 is supplied into blood purification instrument 120. In dialysis fluid pipe channel 141, first valve 141v that opens and closes dialysis fluid pipe channel 141 is provided upstream of a position of connection to first connection pipe channel 132. In dialysis fluid pipe channel 141, first heater 170 that heats the dialysis fluid is provided downstream from dialysis fluid pump 160. First heater 170 does not have to be provided.

Replacement fluid pump 161 that delivers the replacement fluid is connected to replacement fluid pipe channel 131. Though replacement fluid pump 161 is a peristaltic pump, it may be a roller pump. Replacement fluid pipe channel 131 has an upstream end connected to first connection pipe channel 132 through which first fluid 10 flows. In replacement fluid pipe channel 131, second heater 171 that heats the replacement fluid is provided downstream from replacement fluid pump 161. Second heater 171 does not have to be provided.

In the present embodiment, replacement fluid pipe channel 131 is connected to vein-side blood circuit 116. Specifically, replacement fluid pipe channel 131 is connected to vein-side air trap chamber 114. The replacement fluid that has flowed through replacement fluid pipe channel 131 is supplied into vein-side air trap chamber 114. In other words, the replacement fluid is supplied to vein-side blood circuit 116 through replacement fluid pipe channel 131. Blood purification apparatus 100 adopts what is called post dilution. Pre dilution in which replacement fluid pipe channel 131 is connected to artery-side air trap chamber 112 and the replacement fluid is supplied to artery-side blood circuit 110 through replacement fluid pipe channel 131 may be adopted.

First connection pipe channel 132 is connected to first fluid storage portion 140 in which first fluid 10 is temporarily stored and from which stored first fluid 10 can be delivered. Second valve 132v that opens and closes first connection pipe channel 132 is provided in first connection pipe channel 132.

A waste fluid discharged from blood purification instrument 120 is discharged from a downstream end of waste fluid pipe channel 151. Waste fluid pump 162 that delivers the waste fluid that flows through waste fluid pipe channel 151 is connected to waste fluid pipe channel 151. Though waste fluid pump 162 is a peristaltic pump, it may be a roller pump. Second connection pipe channel 152 through which the waste fluid flows is connected to waste fluid pipe channel 151. Third valve 151v that opens and closes waste fluid pipe channel 151 is provided in waste fluid pipe channel 151.

Second connection pipe channel 152 is connected to second fluid storage portion 150 in which the waste fluid is temporarily stored and from which the stored waste fluid can be delivered. Fourth valve 152v that opens and closes second connection pipe channel 152 is provided in second connection pipe channel 152.

First fluid storage portion 140 and second fluid storage portion 150 are accommodated in an accommodation portion 181. Accommodation portion 181 is connected to a measurement unit of scale 180. In the present embodiment, scale 180 is a load cell. Scale 180, however, is not limited to the load cell, but it may be a spring scale or the like. Scale 180 measures weight change of first fluid storage portion 140 and second fluid storage portion 150 as a whole. While fourth valve 152v closes second connection pipe channel 152, scale 180 can measure weight change of first fluid storage portion 140.

Priming fluid pipe channel 191 is connected between artery-side on-off valve 110v and blood pump 111 in artery-side blood circuit 110 and a priming fluid 20 is supplied therethrough. Fifth valve 191v that opens and closes priming fluid pipe channel 191 is provided in priming fluid pipe channel 191. Priming fluid 20 is a physiological saline or the like.

Artery-side gas supply and exhaust pipe channel 117 is connected between artery-side pressure measurement apparatus 113 and artery-side protective filter 117f. Sixth valve 117v that opens and closes artery-side gas supply and exhaust pipe channel 117 is provided in artery-side gas supply and exhaust pipe channel 117. Artery-side gas supply and exhaust pipe channel 117 is configured for exhaust of air in artery-side air trap chamber 112 and supply of air into artery-side air trap chamber 112.

Vein-side gas supply and exhaust pipe channel 118 is connected between vein-side pressure measurement apparatus 115 and vein-side protective filter 118f. In vein-side gas supply and exhaust pipe channel 118, seventh valve 118v that opens and closes vein-side gas supply and exhaust pipe channel 118 is provided. Vein-side gas supply and exhaust pipe channel 118 is configured for exhaust of air in vein-side air trap chamber 114 and supply of air into vein-side air trap chamber 114.

Each of artery-side protective filter 117f and vein-side protective filter 118f performs a function to prevent blood from flowing out of the blood circuit and a function to prevent infection from the pressure measurement apparatus.

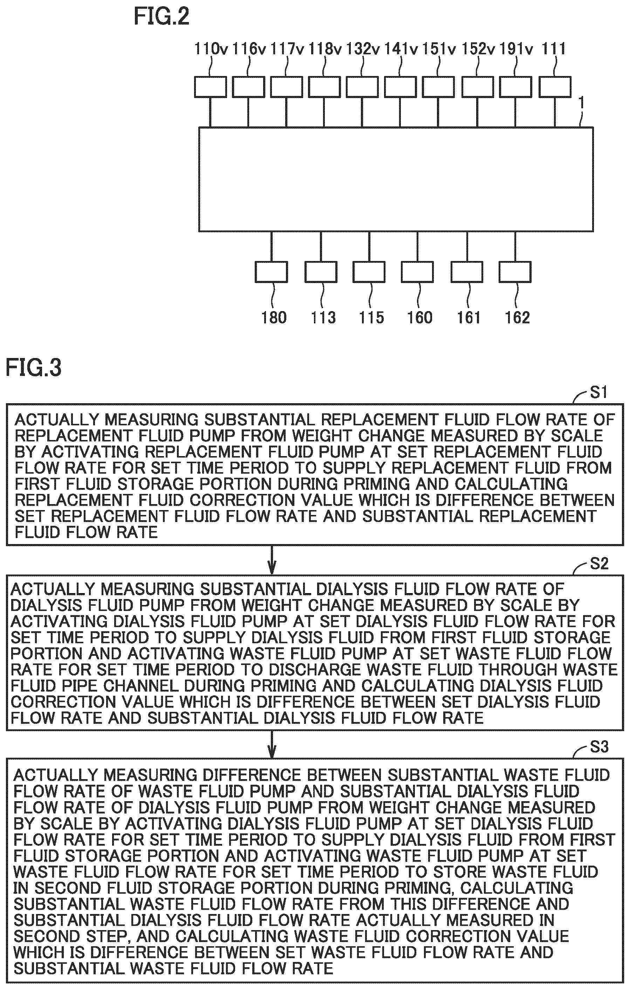

Fig. 2 is a block diagram showing relation of electrical connection between the controller and each component in the blood purification apparatus according to one embodiment of the present invention. As shown in Fig. 2, a controller 1 included in blood purification apparatus 100 is electrically connected to each of blood pump 111, artery-side on-off valve 110v, vein-side on-off valve 116v, first valve 141v, second valve 132v, third valve 151v, fourth valve 152v, fifth valve 191v, sixth valve 117v, seventh valve 118v, artery-side pressure measurement apparatus 113, vein-side pressure measurement apparatus 115, dialysis fluid pump 160, replacement fluid pump 161, waste fluid pump 162, and scale 180.

A measurement value of weight change of first fluid storage portion 140 and second fluid storage portion 150 as a whole measured by scale 180 is inputted to controller 1.

Controller 1 controls an operation of each of blood pump 111, artery-side on-off valve 111v, vein-side on-off valve 116v, first valve 141v, second valve 132v, third valve 151v, fourth valve 152v, fifth valve 191v, sixth valve 117v, seventh valve 118v, dialysis fluid pump 160, replacement fluid pump 161, and waste fluid pump 162.

An operation performed by controller 1 to calculate a correction value of a flow rate of each of dialysis fluid pump 160, replacement fluid pump 161, and waste fluid pump 162 during priming in blood purification apparatus 100 according to the present embodiment will be described below. Fig. 3 is a flowchart showing an operation performed by a controller to calculate a correction value of a flow rate of each of a dialysis fluid pump, a replacement fluid pump, and a waste fluid pump during priming in a blood purification apparatus according to a first embodiment of the present invention.

Initially, a dialysis fluid supply rate, a replacement fluid supply rate, and a filtration rate and a water removal rate in blood purification instrument 120 are set. Then, each of the flow rate of dialysis fluid pump 160, the flow rate of replacement fluid pump 161, and the flow rate of waste fluid pump 162 is set to satisfy each of the dialysis fluid supply rate, the replacement fluid supply rate, and the filtration rate and the water removal rate that have been set. These set flow rates are referred to as a set dialysis fluid flow rate, a set replacement fluid flow rate, and a set waste fluid flow rate, respectively.

Then, third valve 151v and fourth valve 152v are closed and first valve 141v and second valve 132v are opened. First fluid 10 thus flows into first connection pipe channel 132. Some of first fluid 10 that has flowed into first connection pipe channel 132 flows into first fluid storage portion 140 owing to a pressure resulting from a height difference in arrangement of first supply source 130 and first fluid storage portion 140 and is stored therein. Thereafter, first valve 141v is closed.

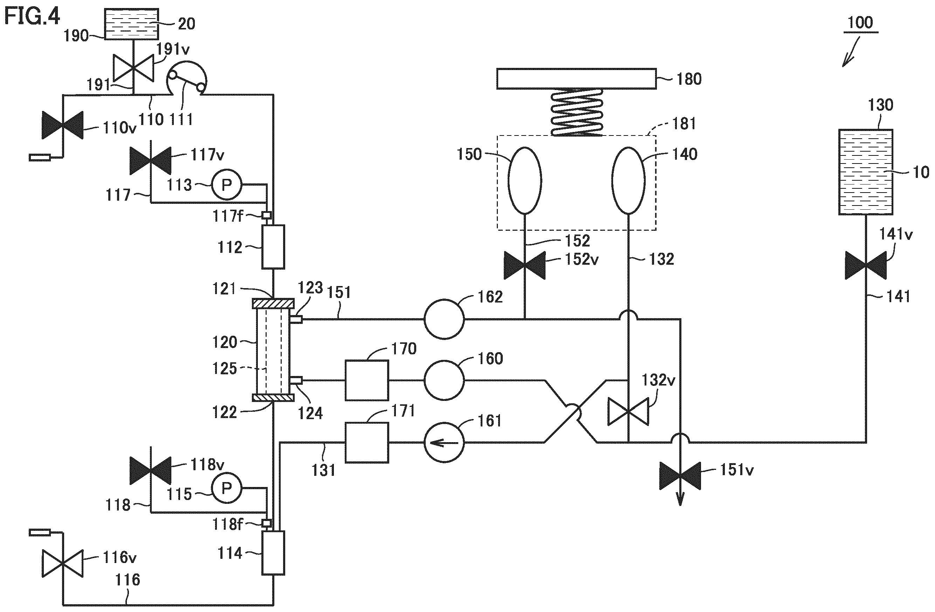

The controller then performs a first step (S1) as shown in Fig. 3. Fig. 4 is a circuit diagram showing a state in which the first step is performed in the blood purification apparatus according to the first embodiment of the present invention.

As shown in Figs. 3 and 4, in the first step (S1), controller 1 activates replacement fluid pump 161 at the set replacement fluid flow rate for a set time period to supply first fluid 10 which is the replacement fluid from first fluid storage portion 140 while first fluid 10 which is the replacement fluid is stored in first fluid storage portion 140 during priming in which artery-side on-off valve 110v is closed and priming fluid 20 is supplied from priming fluid pipe channel 191 to artery-side blood circuit 110, blood purification instrument 120, and vein-side blood circuit 116. A substantial replacement fluid flow rate of replacement fluid pump 161 is thus actually measured from weight change measured by scale 180. Controller 1 calculates a replacement fluid correction value which is a difference between the set replacement fluid flow rate and the substantial replacement fluid flow rate. The calculated replacement fluid correction value is given as feedback on the set replacement fluid flow rate of replacement fluid pump 161 at the time of start of therapy such that there is no error between the set replacement fluid flow rate and the substantial replacement fluid flow rate.

After the first step (S1), first valve 141v is opened and replacement fluid pump 161 is deactivated. Some of first fluid 10 that has flowed into first connection pipe channel 132 thus flows into first fluid storage portion 140 owing to the pressure resulting from the height difference in arrangement of first supply source 130 and first fluid storage portion 140 and is stored therein. Thereafter, first valve 141v is closed and third valve 151v is opened.

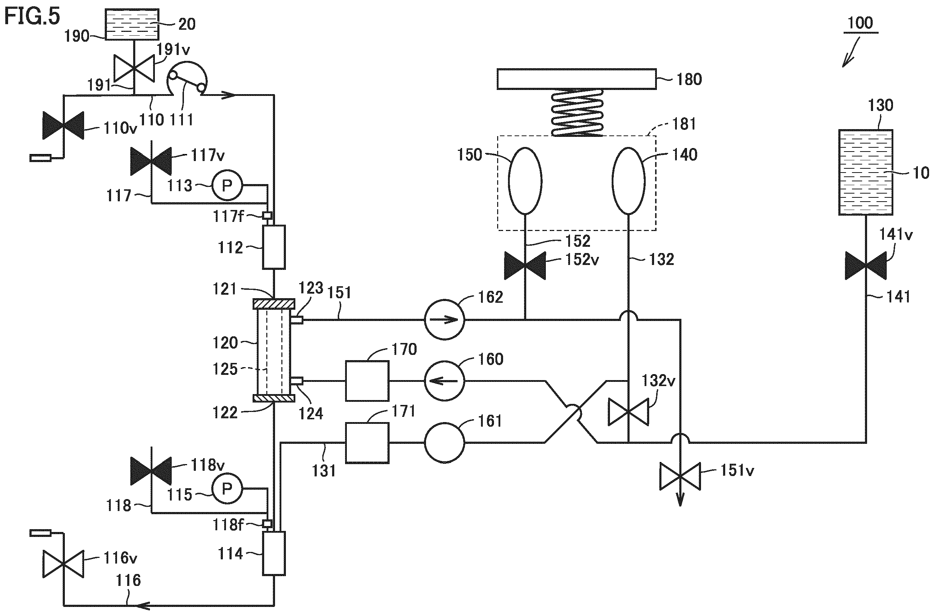

The controller then performs a second step (S2) as shown in Fig. 3. Fig. 5 is a circuit diagram showing a state in which the second step is performed in the blood purification apparatus according to the first embodiment of the present invention.

As shown in Figs. 3 and 5, in the second step (S2), controller 1 activates dialysis fluid pump 160 at the set dialysis fluid flow rate for a set time period to supply first fluid 10 which is the dialysis fluid from first fluid storage portion 140 while first fluid 10 which is the dialysis fluid is stored in first fluid storage portion 140 and activates waste fluid pump 162 at the set waste fluid flow rate for this set time period to discharge the waste fluid through waste fluid pipe channel 151 during priming. A substantial dialysis fluid flow rate of dialysis fluid pump 160 is thus actually measured from weight change measured by scale 180. Controller 1 calculates a dialysis fluid correction value which is a difference between the set dialysis fluid flow rate and the substantial dialysis fluid flow rate. The calculated dialysis fluid correction value is given as feedback on the set dialysis fluid flow rate of dialysis fluid pump 160 at the time of start of therapy such that there is no error between the set dialysis fluid flow rate and the substantial dialysis fluid flow rate.

In the present embodiment, when the controller performs the second step (S2), it activates blood pump 111 to supply priming fluid 20 to blood purification instrument 120. Suction of air by waste fluid pump 162 when the flow rate of waste fluid pump 162 is higher than the flow rate of dialysis fluid pump 160 can thus be suppressed.

After the second step (S2), first valve 141v is opened, third valve 151v is closed, and blood pump 111, dialysis fluid pump 160, and waste fluid pump 162 are deactivated. Some of first fluid 10 that has flowed into first connection pipe channel 132 thus flows into first fluid storage portion 140 owing to the pressure resulting from the height difference in arrangement of first supply source 130 and first fluid storage portion 140 and is stored therein. Thereafter, first valve 141v is closed and third valve 151v and fourth valve 152v are opened. When second fluid storage portion 150 becomes empty, third valve 151v is closed.

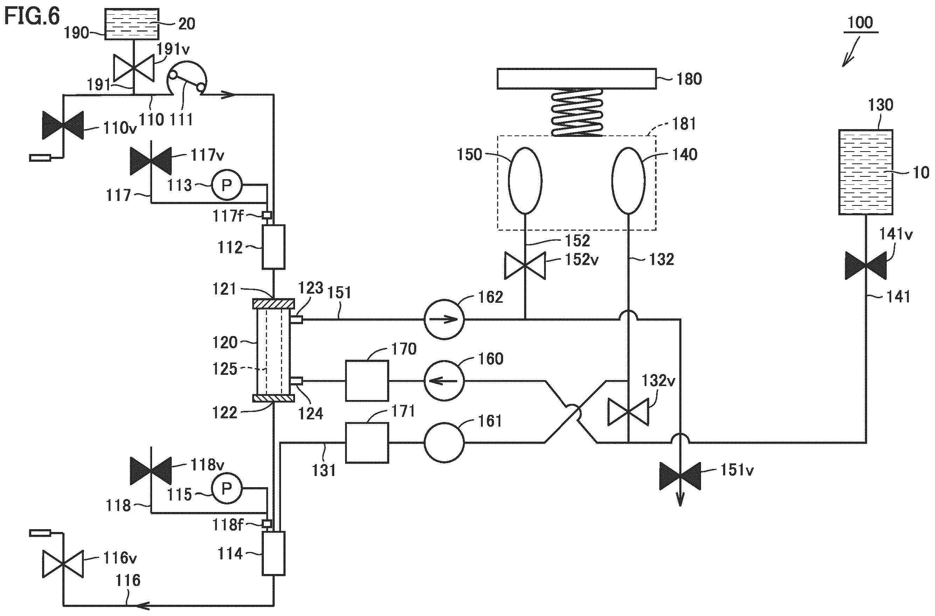

The controller then performs a third step (S3) as shown in Fig. 3. Fig. 6 is a circuit diagram showing a state in which the third step is performed in the blood purification apparatus according to the first embodiment of the present invention.

As shown in Figs. 3 and 6, in the third step (S3), controller 1 activates dialysis fluid pump 160 at the set dialysis fluid flow rate for a set time period to supply the dialysis fluid from first fluid storage portion 140 while first fluid 10 which is the dialysis fluid is stored in first fluid storage portion 140 and activates waste fluid pump 162 at the set waste fluid flow rate for this set time period to store the waste fluid in second fluid storage portion 150 during priming. A difference between the substantial waste fluid flow rate of waste fluid pump 162 and the substantial dialysis fluid flow rate of dialysis fluid pump 160 is thus actually measured from weight change measured by scale 180. Controller 1 calculates the substantial waste fluid flow rate from this difference and the substantial dialysis fluid flow rate actually measured in the second step. Furthermore, controller 1 calculates a waste fluid correction value which is a difference between the set waste fluid flow rate and the substantial waste fluid flow rate. The calculated waste fluid correction value is given as feedback on the set waste fluid flow rate of waste fluid pump 162 at the time of start of therapy such that there is no error between the set waste fluid flow rate and the substantial waste fluid flow rate.

In the present embodiment, when the controller performs the third step (S3), it activates blood pump 111 to supply priming fluid 20 to blood purification instrument 120. Suction of air by waste fluid pump 162 when the flow rate of waste fluid pump 162 is higher than the flow rate of dialysis fluid pump 160 can thus be suppressed.

In blood purification apparatus 100 according to the present embodiment, as set forth above, controller 1 calculates and stores the replacement fluid correction value, the dialysis fluid correction value, and the waste fluid correction value during priming before start of therapy and gives these correction values as feedback at the time of start of therapy, so that each of replacement fluid pump 161, dialysis fluid pump 160, and waste fluid pump 162 can be driven at an accurate flow rate at the time of start of therapy.

If an obviously abnormal value of any one of the replacement fluid correction value, the dialysis fluid correction value, and the waste fluid correction value is calculated, determination as abnormality in a circuit of blood purification apparatus 100 can be made before start of therapy. Furthermore, by performing the first step to the third step by using the physiological saline rather than a therapy fluid, the flow rate of each of replacement fluid pump 161, dialysis fluid pump 160, and waste fluid pump 162 can inexpensively be corrected.

Blood purification apparatuses according to a first modification and a second modification different from the blood purification apparatus according to the first embodiment of the present invention only in operation in the third step (S3) will now be described.

In the blood purification apparatus according to the first modification, in the third step (S3), controller 1 actually measures the substantial waste fluid flow rate of waste fluid pump 162 from weight change measured by scale 180 by activating dialysis fluid pump 160 at the set dialysis fluid flow rate for the set time period to supply first fluid 10 which is the dialysis fluid through dialysis fluid pipe channel 141 without going through first fluid storage portion 140 and activating waste fluid pump 162 at the set waste fluid flow rate for this set time period to store the waste fluid in second fluid storage portion 150 during priming and calculates the waste fluid correction value which is the difference between the set waste fluid flow rate and the substantial waste fluid flow rate. In other words, in the first modification, in performing the third step (S3), first valve 141v is opened and second valve 132v is closed. In the blood purification apparatus according to the first modification, the waste fluid correction value can be calculated based on the actually measured substantial waste fluid flow rate.

In the blood purification apparatus according to the second modification, in the third step (S3), controller 1 actually measures the substantial waste fluid flow rate of waste fluid pump 162 from weight change measured by scale 180 by activating blood pump 111 to discharge the waste fluid from blood purification instrument 120 to waste fluid pipe channel 151 and activating waste fluid pump 162 at the set waste fluid flow rate for this set time period to store the waste fluid in second fluid storage portion 150 during priming and calculates the waste fluid correction value which is the difference between the set waste fluid flow rate and the substantial waste fluid flow rate. In other words, in the second modification, dialysis fluid pump 160 is not active when the third step (S3) is performed. In the blood purification apparatus according to the second modification, the waste fluid correction value can be calculated based on the actually measured substantial waste fluid flow rate.

### (Second Embodiment)

A blood purification apparatus according to a second embodiment of the present invention will be described below with reference to the drawings. Since the blood purification apparatus according to the second embodiment of the present invention is different from the blood purification apparatus according to the first embodiment only in operation in each of the steps for calculating the replacement fluid correction value, the dialysis fluid correction value, and the waste fluid correction value, description of a feature similar to that in blood purification apparatus 100 according to the first embodiment will not be repeated.

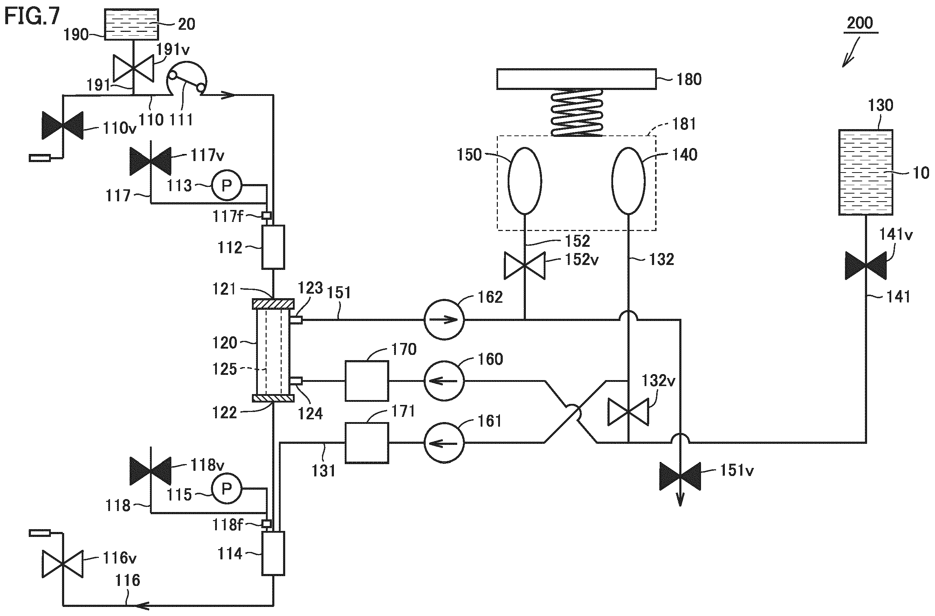

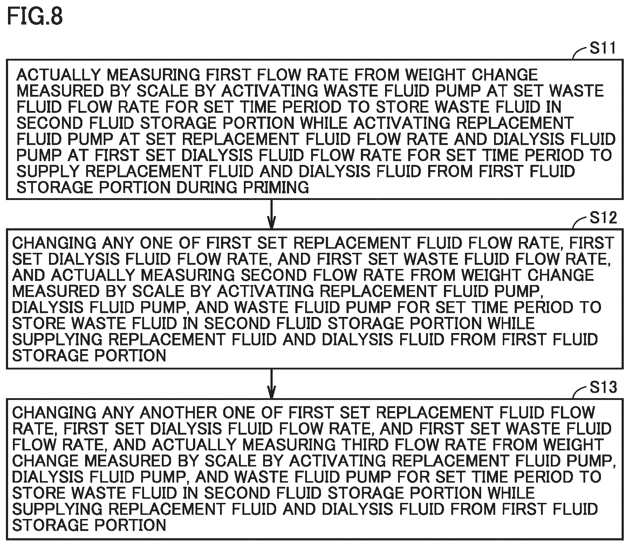

Fig. 7 is a circuit diagram showing a state in which each of the first step to the third step is performed in the blood purification apparatus according to the second embodiment of the present invention. Fig. 8 is a flowchart showing an operation performed by the controller to calculate a correction value of the flow rate of each of the dialysis fluid pump, the replacement fluid pump, and the waste fluid pump during priming in the blood purification apparatus according to the second embodiment of the present invention.

An operation performed by controller 1 to calculate the correction value of the flow rate of each of dialysis fluid pump 160, replacement fluid pump 161, and waste fluid pump 162 during priming in a blood purification apparatus 200 according to the present embodiment will be described.

Initially, third valve 151v and fourth valve 152v are closed and first valve 141v and second valve 132v are opened. First fluid 10 thus flows into first connection pipe channel 132. Some of first fluid 10 that has flowed into first connection pipe channel 132 flows into first fluid storage portion 140 owing to the pressure resulting from the height difference in arrangement of first supply source 130 and first fluid storage portion 140 and is stored therein. First valve 141v is thereafter closed.

Then, as shown in Figs. 8 and 7, in the first step (S11), controller 1 activates waste fluid pump 162 at the set waste fluid flow rate for the set time period to store the waste fluid in second fluid storage portion 150 while it activates replacement fluid pump 161 at the set replacement fluid flow rate and dialysis fluid pump 160 at the set dialysis fluid flow rate for the set time period to supply first fluid 10 which is the replacement fluid and the dialysis fluid from first fluid storage portion 140 while first fluid 10 which is the replacement fluid and the dialysis fluid is stored in first fluid storage portion 140 during priming in which artery-side on-off valve 110v is closed and priming fluid 20 is supplied from priming fluid pipe channel 191 to artery-side blood circuit 110, blood purification instrument 120 and vein-side blood circuit 116. A first flow rate is thus actually measured from weight change measured by scale 180.

Relation of WSa = FP-SFP-DP is satisfied, where WSa represents the first flow rate, SFP represents the substantial replacement fluid flow rate, DP represents the substantial dialysis fluid flow rate, and FP represents the substantial waste fluid flow rate.

Then, as shown in Figs. 8 and 7, in the second step (S12), controller 1 changes any one of the set replacement fluid flow rate, the set dialysis fluid flow rate, and the set waste fluid flow rate that have been set in the first step (S11), and activates replacement fluid pump 161, dialysis fluid pump 160, and waste fluid pump 162 for the set time period to store the waste fluid in second fluid storage portion 150 while supplying first fluid 10 which is the replacement fluid and the dialysis fluid from first fluid storage portion 140. A second flow rate is thus actually measured from weight change measured by scale 180. In the present embodiment, in the second step (S12), the set waste fluid flow rate is changed to 1.2 time higher to set the substantial waste fluid flow rate to 1.2FP. Relation of WSb = 1.2FP-SFP-DP is satisfied, where WSb represents the second flow rate.

Then, as shown in Figs. 8 and 7, in the third step (S13), controller 1 changes any another one of the set replacement fluid flow rate, the set dialysis fluid flow rate, and the set waste fluid flow rate that have been set in the first step (S11), and activates replacement fluid pump 161, dialysis fluid pump 160, and waste fluid pump 162 for the set time period to store the waste fluid in second fluid storage portion 150 while supplying first fluid 10 which is the replacement fluid and the dialysis fluid from first fluid storage portion 140. A third flow rate is thus actually measured from weight change measured by scale 180. In the present embodiment, in the third step (S13), the set replacement fluid flow rate is changed to 1.2 time higher to set the substantial replacement fluid flow rate to 1.2SFP. Relation of WSc = FP-1.2SFP-DP is satisfied, where WSc represents the third flow rate.

Controller 1 calculates a first correction value which is a difference between a first substantial flow rate and the any one of the set replacement fluid flow rate, the set dialysis fluid flow rate, and the set waste fluid flow rate, the first substantial flow rate being calculated by dividing a difference calculated by subtracting first flow rate WSa from second flow rate WSb by a rate of change in the any one of the set replacement fluid flow rate, the set dialysis fluid flow rate, and the set waste fluid flow rate. In the present embodiment, controller 1 calculates the substantial waste fluid flow rate which is the first substantial flow rate from a relational expression FP = (WSb-WSa)/0.2 derived from a relational expression WSb-WSa = 0.2FP. Controller 1 calculates the first correction value which is a difference between the set waste fluid flow rate and the substantial waste fluid flow rate. The calculated first correction value is given as feedback on the set waste fluid flow rate of waste fluid pump 162 at the time of start of therapy such that there is no error between the set waste fluid flow rate and the substantial waste fluid flow rate.

Controller 1 calculates a second correction value which is a difference between a second substantial flow rate and the any another one of the set replacement fluid flow rate, the set dialysis fluid flow rate, and the set waste fluid flow rate, the second substantial flow rate being calculated by dividing a difference calculated by subtracting first flow rate WSa from third flow rate WSc by a rate of change in the any another one of the set replacement fluid flow rate, the set dialysis fluid flow rate, and the set waste fluid flow rate. In the present embodiment, controller 1 calculates the substantial replacement fluid flow rate which is the second substantial flow rate from a relational expression SFP = (WSc-WSa)/(-0.2) derived from a relational expression WSc-WSa = -0.2SFP. Controller 1 calculates the second correction value which is a difference between the set replacement fluid flow rate and the substantial replacement fluid flow rate. The calculated second correction value is given as feedback on the set replacement fluid flow rate of dialysis fluid pump 160 at the time of start of therapy such that there is no error between the set replacement fluid flow rate and the substantial replacement fluid flow rate.

Controller 1 calculates a third correction value which is a difference between a third substantial flow rate and remaining one of the set replacement fluid flow rate, the set dialysis fluid flow rate, and the set waste fluid flow rate that have been set in the first step (S11), the third substantial flow rate being calculated from the relational expression of first flow rate WSa with the first substantial flow rate and the second substantial flow rate. In the present embodiment, controller 1 calculates the substantial dialysis fluid flow rate which is the third substantial flow rate from a relational expression DP = FP-SFP-WSa. Controller 1 calculates the third correction value which is a difference between the set dialysis fluid flow rate and the substantial dialysis fluid flow rate. The calculated third correction value is given as feedback on the set dialysis fluid flow rate of dialysis fluid pump 160 at the time of start of therapy such that there is no error between the set dialysis fluid flow rate and the substantial dialysis fluid flow rate.

In the present embodiment, when controller 1 performs the first step (S11) to the third step (S13), it activates blood pump 111 to supply priming fluid 20 to blood purification instrument 120. Suction of air by waste fluid pump 162 when the flow rate of waste fluid pump 162 is higher than the flow rate of dialysis fluid pump 160 can thus be suppressed.

In blood purification apparatus 200 according to the present embodiment, as set forth above, controller 1 calculates and stores the first correction value, the second fluid correction value, and the third correction value during priming before start of therapy and gives these correction values as feedback at the time of start of therapy, so that each of replacement fluid pump 161, dialysis fluid pump 160, and waste fluid pump 162 can be driven at an accurate flow rate at the time of start of therapy.

If an obviously abnormal value of any one of the first correction value, the second fluid correction value, and the third correction value is calculated, determination as abnormality in a circuit of blood purification apparatus 200 can be made before start of therapy. Furthermore, by performing the first step to the third step by using the physiological saline rather than a therapy fluid, the flow rate of each of replacement fluid pump 161, dialysis fluid pump 160, and waste fluid pump 162 can inexpensively be corrected.

The set dialysis fluid flow rate may be changed in the third step (S13) or the set dialysis fluid flow rate may be changed in the second step (S12).

Though the configuration in which the replacement fluid and the dialysis fluid are supplied from first supply source 130 is illustrated in the description of the embodiments above, without being limited as such, the replacement fluid and the dialysis fluid may be supplied from supply sources different from each other. In this case, replacement fluid pipe channel 131 is configured to be connected to a replacement fluid supply source, dialysis fluid pipe channel 141 is configured to be connected to a dialysis fluid supply source, and first fluid storage portion 140 is configured to include two fluid storage portions which are a replacement fluid storage portion connected to replacement fluid pipe channel 131 and a dialysis fluid storage portion connected to dialysis fluid pipe channel 141.

It should be understood that the embodiments disclosed herein are illustrative and non-restrictive in every respect. The scope of the present invention is defined by the terms of the claims rather than the description above and is intended to include any modifications within the scope and meaning equivalent to the terms of the claims.

### REFERENCE SIGNS LIST

1 controller; 10 first fluid; 20 priming fluid; 100, 200 blood purification apparatus; 110 artery-side blood circuit; 110v artery-side on-off valve; 111 blood pump; 112 artery-side air trap chamber; 113 artery-side pressure measurement apparatus; 114 vein-side air trap chamber; 115 vein-side pressure measurement apparatus; 116 vein-side blood circuit; 116v vein-side on-off valve; 117 artery-side gas supply and exhaust pipe channel; 117f artery-side protective filter; 117v sixth valve; 118 vein-side gas supply and exhaust pipe channel; 118f vein-side protective filter; 118v seventh valve; 120 blood purification instrument; 121 blood inlet; 122 blood outlet; 123 waste fluid outlet; 124 dialysis fluid inlet; 130 first supply source; 131 replacement fluid pipe channel; 131v second valve; 132 first connection pipe channel; 140 first fluid storage portion; 141 dialysis fluid pipe channel; 141v first valve; 150 second fluid storage portion; 151 waste fluid pipe channel; 151v third valve; 152 second connection pipe channel; 152v fourth valve; 160 dialysis fluid pump; 161 replacement fluid pump; 162 waste fluid pump; 170 first heater; 171 second heater; 180 scale; 181 accommodation portion; 190 priming fluid supply source; 191 priming fluid pipe channel; 191v fifth valve.

## Claims

1. A blood purification apparatus comprising:
a blood purification instrument;
an artery-side blood circuit connected to the blood purification instrument, the artery-side blood circuit being configured for inflow of blood into the blood purification instrument;
a vein-side blood circuit connected to the blood purification instrument, the vein-side blood circuit being configured for outflow of blood from the blood purification instrument;
a replacement fluid pipe channel connected to the artery-side blood circuit or the vein-side blood circuit, a replacement fluid being supplied through the replacement fluid pipe channel;
a dialysis fluid pipe channel through which a dialysis fluid is supplied to the blood purification instrument;
a waste fluid pipe channel through which a waste fluid discharged from the blood purification instrument flows;
a blood pump provided in the artery-side blood circuit, the blood pump being configured to deliver blood;
a replacement fluid pump provided in the replacement fluid pipe channel, the replacement fluid pump being configured to deliver the replacement fluid;
a dialysis fluid pump provided in the dialysis fluid pipe channel, the dialysis fluid pump being configured to deliver the dialysis fluid;
a waste fluid pump provided in the waste fluid pipe channel, the waste fluid pump being configured to deliver the waste fluid;
an artery-side on-off valve provided in the artery-side blood circuit, the artery-side on-off valve being configured to open and close the artery-side blood circuit;
a priming fluid pipe channel connected to the artery-side blood circuit between the artery-side on-off valve and the blood pump, a priming fluid being supplied through the priming fluid pipe channel;
a first fluid storage portion in which the replacement fluid and the dialysis fluid are temporarily stored and from which the stored replacement fluid and the stored dialysis fluid can be delivered, the first fluid storage portion being connected to the replacement fluid pipe channel and the dialysis fluid pipe channel;
a second fluid storage portion in which the waste fluid is temporarily stored and from which the stored waste fluid can be delivered, the second fluid storage portion being connected to the waste fluid pipe channel;
a scale that can measure weight change of the first fluid storage portion and the second fluid storage portion as a whole; and
a controller that controls drive of each of the blood pump, the replacement fluid pump, the dialysis fluid pump, and the waste fluid pump, wherein
the controller performs
a first step of actually measuring a substantial replacement fluid flow rate of the replacement fluid pump from weight change measured by the scale by activating the replacement fluid pump at a set replacement fluid flow rate for a set time period to supply the replacement fluid from the first fluid storage portion while the replacement fluid is stored in the first fluid storage portion during priming in which the artery-side on-off valve is closed and the priming fluid is supplied from the priming fluid pipe channel to the artery-side blood circuit, the blood purification instrument, and the vein-side blood circuit, and calculating a replacement fluid correction value which is a difference between the set replacement fluid flow rate and the substantial replacement fluid flow rate,
a second step of actually measuring a substantial dialysis fluid flow rate of the dialysis fluid pump from weight change measured by the scale by activating the dialysis fluid pump at a set dialysis fluid flow rate for a set time period to supply the dialysis fluid from the first fluid storage portion while the dialysis fluid is stored in the first fluid storage portion and activating the waste fluid pump at a set waste fluid flow rate for the set time period to discharge the waste fluid through the waste fluid pipe channel during the priming, and calculating a dialysis fluid correction value which is a difference between the set dialysis fluid flow rate and the substantial dialysis fluid flow rate, and
a third step of actually measuring a difference between a substantial waste fluid flow rate of the waste fluid pump and the substantial dialysis fluid flow rate of the dialysis fluid pump from weight change measured by the scale by activating the dialysis fluid pump at the set dialysis fluid flow rate for the set time period to supply the dialysis fluid from the first fluid storage portion while the dialysis fluid is stored in the first fluid storage portion and activating the waste fluid pump at a set waste fluid flow rate for the set time period to store the waste fluid in the second fluid storage portion during the priming, calculating the substantial waste fluid flow rate from the difference and the substantial dialysis fluid flow rate actually measured in the second step, and calculating a waste fluid correction value which is a difference between the set waste fluid flow rate and the substantial waste fluid flow rate.

2. A blood purification apparatus comprising:
a blood purification instrument;
an artery-side blood circuit connected to the blood purification instrument, the artery-side blood circuit being configured for inflow of blood into the blood purification instrument;
a vein-side blood circuit connected to the blood purification instrument, the vein-side blood circuit being configured for outflow of blood from the blood purification instrument;
a replacement fluid pipe channel connected to the artery-side blood circuit or the vein-side blood circuit, a replacement fluid being supplied through the replacement fluid pipe channel;
a dialysis fluid pipe channel through which a dialysis fluid is supplied to the blood purification instrument;
a waste fluid pipe channel through which a waste fluid discharged from the blood purification instrument flows;
a blood pump provided in the artery-side blood circuit, the blood pump being configured to deliver blood;
a replacement fluid pump provided in the replacement fluid pipe channel, the replacement fluid pump being configured to deliver the replacement fluid;
a dialysis fluid pump provided in the dialysis fluid pipe channel, the dialysis fluid pump being configured to deliver the dialysis fluid;
a waste fluid pump provided in the waste fluid pipe channel, the waste fluid pump being configured to deliver the waste fluid;
an artery-side on-off valve provided in the artery-side blood circuit, the artery-side on-off valve being configured to open and close the artery-side blood circuit;
a priming fluid pipe channel connected to the artery-side blood circuit between the artery-side on-off valve and the blood pump, a priming fluid being supplied through the priming fluid pipe channel;
a first fluid storage portion in which the replacement fluid and the dialysis fluid are temporarily stored and from which the stored replacement fluid and the stored dialysis fluid can be delivered, the first fluid storage portion being connected to the replacement fluid pipe channel and the dialysis fluid pipe channel;
a second fluid storage portion in which the waste fluid is temporarily stored and from which the stored waste fluid can be delivered, the second fluid storage portion being connected to the waste fluid pipe channel;
a scale that can measure weight change of the first fluid storage portion and the second fluid storage portion as a whole; and
a controller that controls drive of each of the blood pump, the replacement fluid pump, the dialysis fluid pump, and the waste fluid pump, wherein
the controller performs
a first step of actually measuring a substantial replacement fluid flow rate of the replacement fluid pump from weight change measured by the scale by activating the replacement fluid pump at a set replacement fluid flow rate for a set time period to supply the replacement fluid from the first fluid storage portion while the replacement fluid is stored in the first fluid storage portion during priming in which the artery-side on-off valve is closed and the priming fluid is supplied from the priming fluid pipe channel to the artery-side blood circuit, the blood purification instrument, and the vein-side blood circuit, and calculating a replacement fluid correction value which is a difference between the set replacement fluid flow rate and the substantial replacement fluid flow rate,
a second step of actually measuring a substantial dialysis fluid flow rate of the dialysis fluid pump from weight change measured by the scale by activating the dialysis fluid pump at a set dialysis fluid flow rate for a set time period to supply the dialysis fluid from the first fluid storage portion while the dialysis fluid is stored in the first fluid storage portion and activating the waste fluid pump at a set waste fluid flow rate for the set time period to discharge the waste fluid through the waste fluid pipe channel during the priming, and calculating a dialysis fluid correction value which is a difference between the set dialysis fluid flow rate and the substantial dialysis fluid flow rate, and
a third step of actually measuring a substantial waste fluid flow rate of the waste fluid pump from weight change measured by the scale by activating the dialysis fluid pump at the set dialysis fluid flow rate for the set time period to supply the dialysis fluid through the dialysis fluid pipe channel without going through the first fluid storage portion and activating the waste fluid pump at a set waste fluid flow rate for the set time period to store the waste fluid in the second fluid storage portion during the priming, and calculating a waste fluid correction value which is a difference between the set waste fluid flow rate and the substantial waste fluid flow rate.

3. The blood purification apparatus according to claim 1 or 2, wherein
the controller activates the blood pump to supply the priming fluid to the blood purification instrument when the controller performs the second step and the third step.

4. A blood purification apparatus comprising:
a blood purification instrument;
an artery-side blood circuit connected to the blood purification instrument, the artery-side blood circuit being configured for inflow of blood into the blood purification instrument;
a vein-side blood circuit connected to the blood purification instrument, the vein-side blood circuit being configured for outflow of blood from the blood purification instrument;
a replacement fluid pipe channel connected to the artery-side blood circuit or the vein-side blood circuit, a replacement fluid being supplied through the replacement fluid pipe channel;
a dialysis fluid pipe channel through which a dialysis fluid is supplied to the blood purification instrument;
a waste fluid pipe channel through which a waste fluid discharged from the blood purification instrument flows;
a blood pump provided in the artery-side blood circuit, the blood pump being configured to deliver blood;
a replacement fluid pump provided in the replacement fluid pipe channel, the replacement fluid pump being configured to deliver the replacement fluid;
a dialysis fluid pump provided in the dialysis fluid pipe channel, the dialysis fluid pump being configured to deliver the dialysis fluid;
a waste fluid pump provided in the waste fluid pipe channel, the waste fluid pump being configured to deliver the waste fluid;
an artery-side on-off valve provided in the artery-side blood circuit, the artery-side on-off valve being configured to open and close the artery-side blood circuit;
a priming fluid pipe channel connected to the artery-side blood circuit between the artery-side on-off valve and the blood pump, a priming fluid being supplied through the priming fluid pipe channel;
a first fluid storage portion in which the replacement fluid and the dialysis fluid are temporarily stored and from which the stored replacement fluid and the stored dialysis fluid can be delivered, the first fluid storage portion being connected to the replacement fluid pipe channel and the dialysis fluid pipe channel;
a second fluid storage portion in which the waste fluid is temporarily stored and from which the stored waste fluid can be delivered, the second fluid storage portion being connected to the waste fluid pipe channel;
a scale that can measure weight change of the first fluid storage portion and the second fluid storage portion as a whole; and
a controller that controls drive of each of the blood pump, the replacement fluid pump, the dialysis fluid pump, and the waste fluid pump, wherein
the controller performs
a first step of actually measuring a substantial replacement fluid flow rate of the replacement fluid pump from weight change measured by the scale by activating the replacement fluid pump at a set replacement fluid flow rate for a set time period to supply the replacement fluid from the first fluid storage portion while the replacement fluid is stored in the first fluid storage portion during priming in which the artery-side on-off valve is closed and the priming fluid is supplied from the priming fluid pipe channel to the artery-side blood circuit, the blood purification instrument, and the vein-side blood circuit, and calculating a replacement fluid correction value which is a difference between the set replacement fluid flow rate and the substantial replacement fluid flow rate,
a second step of actually measuring a substantial dialysis fluid flow rate of the dialysis fluid pump from weight change measured by the scale by activating the dialysis fluid pump at a set dialysis fluid flow rate for a set time period to supply the dialysis fluid from the first fluid storage portion while the dialysis fluid is stored in the first fluid storage portion and activating the waste fluid pump at a set waste fluid flow rate for the set time period to discharge the waste fluid through the waste fluid pipe channel during the priming, and calculating a dialysis fluid correction value which is a difference between the set dialysis fluid flow rate and the substantial dialysis fluid flow rate, and
a third step of actually measuring a substantial waste fluid flow rate of the waste fluid pump from weight change measured by the scale by activating the blood pump to discharge the waste fluid from the blood purification instrument to the waste fluid pipe channel and activating the waste fluid pump at the set waste fluid flow rate for the set time period to store the waste fluid in the second fluid storage portion during the priming, and calculating a waste fluid correction value which is a difference between the set waste fluid flow rate and the substantial waste fluid flow rate.

5. A blood purification apparatus comprising:
a blood purification instrument;
an artery-side blood circuit connected to the blood purification instrument, the artery-side blood circuit being configured for inflow of blood into the blood purification instrument;
a vein-side blood circuit connected to the blood purification instrument, the vein-side blood circuit being configured for outflow of blood from the blood purification instrument;
a replacement fluid pipe channel connected to the artery-side blood circuit or the vein-side blood circuit, a replacement fluid being supplied through the replacement fluid pipe channel;
a dialysis fluid pipe channel through which a dialysis fluid is supplied to the blood purification instrument;
a waste fluid pipe channel through which a waste fluid discharged from the blood purification instrument flows;
a blood pump provided in the artery-side blood circuit, the blood pump being configured to deliver blood;
a replacement fluid pump provided in the replacement fluid pipe channel, the replacement fluid pump being configured to deliver the replacement fluid;
a dialysis fluid pump provided in the dialysis fluid pipe channel, the dialysis fluid pump being configured to deliver the dialysis fluid;
a waste fluid pump provided in the waste fluid pipe channel, the waste fluid pump being configured to deliver the waste fluid;
an artery-side on-off valve provided in the artery-side blood circuit, the artery-side on-off valve being configured to open and close the artery-side blood circuit;
a priming fluid pipe channel connected to the artery-side blood circuit between the artery-side on-off valve and the blood pump, a priming fluid being supplied through the priming fluid pipe channel;
a first fluid storage portion in which the replacement fluid and the dialysis fluid are temporarily stored and from which the stored replacement fluid and the stored dialysis fluid can be delivered, the first fluid storage portion being connected to the replacement fluid pipe channel and the dialysis fluid pipe channel;
a second fluid storage portion in which the waste fluid is temporarily stored and from which the stored waste fluid can be delivered, the second fluid storage portion being connected to the waste fluid pipe channel;
a scale that can measure weight change of the first fluid storage portion and the second fluid storage portion as a whole; and
a controller that controls drive of each of the blood pump, the replacement fluid pump, the dialysis fluid pump, and the waste fluid pump, wherein
the controller
performs a first step of actually measuring a first flow rate from weight change measured by the scale by activating the waste fluid pump at a set waste fluid flow rate for a set time period to store the waste fluid in the second fluid storage portion while activating the replacement fluid pump at a set replacement fluid flow rate and the dialysis fluid pump at a set dialysis fluid flow rate for the set time period to supply the replacement fluid and the dialysis fluid from the first fluid storage portion while the replacement fluid and the dialysis fluid are stored in the first fluid storage portion during priming in which the artery-side on-off valve is closed and the priming fluid is supplied from the priming fluid pipe channel to the artery-side blood circuit, the blood purification instrument, and the vein-side blood circuit,
performs a second step of changing any one of the set replacement fluid flow rate, the set dialysis fluid flow rate, and the set waste fluid flow rate that have been set in the first step and actually measuring a second flow rate from weight change measured by the scale by activating the replacement fluid pump, the dialysis fluid pump, and the waste fluid pump for the set time period to store the waste fluid in the second fluid storage portion while supplying the replacement fluid and the dialysis fluid from the first fluid storage portion,
performs a third step of changing any another one of the set replacement fluid flow rate, the set dialysis fluid flow rate, and the set waste fluid flow rate that have been set in the first step and actually measuring a third flow rate from weight change measured by the scale by activating the replacement fluid pump, the dialysis fluid pump, and the waste fluid pump for the set time period to store the waste fluid in the second fluid storage portion while supplying the replacement fluid and the dialysis fluid from the first fluid storage portion,
calculates a first correction value which is a difference between a first substantial flow rate and the any one of the set replacement fluid flow rate, the set dialysis fluid flow rate, and the set waste fluid flow rate, the first substantial flow rate being calculated by dividing a difference calculated by subtracting the first flow rate from the second flow rate by a rate of change in the any one of the set replacement fluid flow rate, the set dialysis fluid flow rate, and the set waste fluid flow rate,
calculates a second correction value which is a difference between a second substantial flow rate and the any another one of the set replacement fluid flow rate, the set dialysis fluid flow rate, and the set waste fluid flow rate, the second substantial flow rate being calculated by dividing a difference calculated by subtracting the first flow rate from the third flow rate by a rate of change in the any another one of the set replacement fluid flow rate, the set dialysis fluid flow rate, and the set waste fluid flow rate, and
calculates a third correction value which is a difference between a third substantial flow rate and remaining one of the set replacement fluid flow rate, the set dialysis fluid flow rate, and the set waste fluid flow rate that have been set in the first step, the third substantial flow rate being calculated in accordance with a relational expression of the first flow rate with the first substantial flow rate and the second substantial flow rate.

6. The blood purification apparatus according to claim 5, wherein
the controller activates the blood pump to supply the priming fluid to the blood purification instrument when the controller performs the first step to the third step.
